# EUROPÄISCHE PATENTSCHRIFT

(11) **EP 2 525 865 B1**
(45) Veröffentlichungstag und Bekanntmachung des Hinweises auf die Patenterteilung: **14.02.2018**
(21) Anmeldenummer: 11707357.7
(22) Anmeldetag: 24.01.2011
(51) Int. Cl.: A61M 39/00, A61M 39/22, A61M 39/10

(54) **FLUIDDURCHSTRÖMTE VERBINDUNGSSYSTEME ZUM EINSATZ IN DER MEDIZIN UND MEDIZINTECHNIK**
CONNECTING SYSTEMS THROUGH WHICH FLUID FLOWS FOR USE IN MEDICINE AND MEDICAL TECHNOLOGY
SYSTÈMES DE LIAISON PARCOURUS PAR UN FLUIDE À UTILISER EN MÉDECINE OU EN TECHNIQUE MÉDICALE

(30) Priorität: 22.01.2010 DE 202010000078 U
(43) Veröffentlichungstag der Anmeldung: 28.11.2012
(73) Patentinhaber: Hopf, Hans-Jürgen, 90513 Zirndorf (DE)
(72) Erfinder: HOPF, Hans-Jürgen, 90513 Zirndorf (DE); KASSAI, Norbert, 90522 Oberasbach (DE); HOPF, Alexander, 90491 Nürnberg (DE); HOPF, Michael, 90513 Zirndorf (DE)
(74) Vertreter: Wittmann, Ernst-Ulrich
(86) Internationale Anmeldenummer: PCT/EP2011/050922
(87) Internationale Veröffentlichungsnummer: WO 2011/089254

(56) Entgegenhaltungen:
- US-A- 4 163 722
- US-A- 5 312 377
- US-A- 5 566 680
- US-A- 5 782 808
- US-A1- 2003 100 858
- US-A1- 2005 033 238
- US-A1- 2008 306 469

## Beschreibung

Die vorliegende Erfindung betrifft fluiddurchströmte Verbindungssysteme, wie sie insbesondere in der Medizin eingesetzt werden.

Zum Beispiel sind im Stand der Technik Dreiwegehähne und Y-Verbinder bekannt und werden insbesondere in der Medizin und Medizintechnik eingesetzt. Hierbei werden sie insbesondere im Infusionsbereich, dem Bereich der künstlichen Ernährung, bei der Transfusion und insbesondere für die Zu- bzw. Überleitung von verschiedenen Durchflussmedien und als sogenanntes "Injektions-Equipment" für die medizinische und pharmazeutische Ausrüstung verwendet. Dreiwegehähne werden ferner auch in medizinischen Systemen verwendet, welche unter anderem aus mehreren Komponenten bestehen. Solche Systeme umfassen unter anderem Schwerkraftinfusionen, Pumpen oder Pumpüberleitungssysteme, Sondennahrungssysteme, Injektionen, Kombinationen hiervon und dergleichen. Ein Dreiwegehahn kann ferner durch die Kombination mit mehreren Dreiwegehähnen zu einer sog. Mehrwegehahnbank oder "Manifold" (Mehrfachverteiler) montiert werden.

Im Stand der Technik sind hierzu eine Vielzahl von Dokumenten bekannt wie beispielsweise die US 5566680 A, die US 2006/089603 A1, die US 3185179 A und die US 6287656 B1. Ferner kennt der Fachmann auch Nicht-Patent-Literatur wie beispielsweise POLYONE: "PolyOne Corporation and Eastman Chemical Company Announce Launch of Edgetek XT Blends Based on Eastman TritanCopolyester", 20090623, 23. Juni 2009, Seiten 1-2, XP007920637, gefunden im Internet: URL:http:/www.polyone.com/en-us/nes/pages/archivedPressRelease.aspx?PRID=7 bzw. EASTMAN: "Eastman Tritan CopolyestereX401", 20080123, 23. Januar 2008, Seiten 1-2, XP007920640, gefunden im Internet: URL:http://ws.eastman.com/P?roductCatalogApps/PageControllers/ProdDatasheet_ PC.aspx?product=71068690 und-POYONE: "edgetek XT High Performance Blends Made with BPA-Free Eastman TritanTM Copolyester", 20090101, 1. Januar 2009, Seiten 1-2, XP007920639, gefunden im Interrnet: URL: http://ww.polyone.com/en-us/docs/Documents/Edgetek_XT_Tritan_ABS_Product_Bulletin_gps.pdf, welche sowohl Verbindungssysteme für die Medizin und Medizintechnik betreffen als auch das Material, welches hierfür typischerweise verwendet wurde.

Die im Stand der Technik bekannten fluiddurchströmte Verbindungssysteme haben jedoch den Nachteil, das diese unter anderem aus Kunststoffen hergestellt werden, bei deren Verarbeitung Weichmacher, wie beispielsweise Bisphenol-A eingesetzt werden. Bei der Verwendung der fluiddurchströmten Fluidssystemen kann jedoch nicht ausgeschlossen werden, dass Teile der Weichmacher an die Flüssigkeit abgegeben werden, wobei bekannt ist, dass diese Weichmachermaterialen zu einer Belastung der körperlichen Gesundheit führen können. Unter anderem werden diesen Weichmachern kanzerogene Wirkungen zugeschrieben.

Aufgabe der vorliegenden Erfindung ist es, fluiddurchströmte Verbindungssysteme bereit zu stellen, bei welchen das mögliche Belastungsrisiko des Patienten durch unerwünschte Begleitstoffe wenigstens reduziert wird.

Die Aufgabe wird durch die Verwendung eines amorphen Copolyesters zur Herstellung der fluiddurchströmten Verbindungssysteme und durch entsprechende Verbindungssysteme, welche wenigstens an den mit dem Fluid in Kontakt tretenden Oberflächen ein entsprechendes Material aufweisen, gelöst. Bevorzugte Ausgestaltungen sind Gegenstand der Unteransprüche.

Gemäß einer ersten besonders bevorzugten Ausführungsform der vorliegenden Erfindung wird ein amorpher Copolyester zur Herstellung eines verriegelbaren Verbindungssystems für fluiddurchströmte Bauteile für die Medizin und Medizintechnik wie insbesondere Infussions- oder Transfussionsschläuche, Mehrwegehähne, Mehrfachverteiler, Injektionsequipment wie Nadeln, Zugänge oder ähnliches und Kombinationen hiervon verwendet, wobei das Verbindungssystem wenigstens eine weibliche, rohrförmige Aufnahme, welche einen inneren Aufnahmeabschnitt und einen äußeren ersten Fixierabschnitt aufweist, und/oder ein männliches, rohrförmiges Steckelement zur Aufnahme in den weiblichen Aufnahmeabschnitt und einen äußeren zweiten Fixierabschnitt, welcher mit dem ersten Fixierabschnitt zur Fixierung des Verbindungssystems zusammenwirkt, aufweist.

Gemäß einer weiteren bevorzugten Ausführungsform umfasst die Erfindung ein Verbindungssystem für fluiddurchströmte Bauteile für die Medizin und Medizintechnik wie insbesondere Infussions- oder Transfussionsschläuche, Mehrwegehähne, Mehrfachverteiler, Injektionsequipment wie Nadeln, Zugänge oder ähnliches und Kombinationen hiervon, mit einer weiblichen, rohrförmigen Aufnahme, welche einen inneren Aufnahmeabschnitt und einen äußeren ersten Fixierabschnitt aufweist, und/oder einem männlichen, rohrförmigen Steckelement zur Aufnahme in dem weiblichen Aufnahmeabschnitt und einem äußeren zweiten Fixierabschnitt, welcher mit dem ersten Fixierabschnitt zur Fixierung des Verbindungssystems zusammenwirkt. Dabei ist das System dadurch gekennzeichnet, dass wenigstens die mit dem Fluid in Kontakt kommenden Oberflächen des Verbindungssystems wenigstens abschnittsweise aus einem amorphen Copolyester hergestellt werden.

Ferner werden als Materialien zur Herstellung des erfindungsgemäßen Verbindungssystems neben dem Copolyester weitere Materialien verwendet, welche aus einer Gruppe ausgewählt werden, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen Kombinationen hiervon und dergleichen umfasst.

Die typischerweise in dem Verbindungssystem fließenden Fluide sind aus einer Gruppe ausgewählt, welche Injektionslösungen, Infusionslösungen, Nährlösungen, Blut, Plasma, Gase, Luft Kombinationen hieraus und dergleichen aufweist.

Die vorliegende Erfindung umfasst ferner auch die Verwendung des erfindungsgemäßen Verbindungssystems in der Medizin und/oder Medizintechnik, insbesondere für die Zu- bzw. Überleitung von verschiedenen Flüssigkeiten, insbesondere für die Schwerkraftinfusion, Pumpüberleitungssysteme, Sondennahrungssysteme, Injektionen, Kombinationen hiervon und dergleichen.

Die Erfindung wird nachfolgend anhand eines bevorzugten Ausführungsbeispiels erläutert, wobei darauf hingewiesen wird, dass durch dieses Beispiel Abwandlungen beziehungsweise Ergänzungen wie sie sich für den Fachmann unmittelbar ergeben mit umfasst sind. Darüber hinaus stellt dieses bevorzugte Ausführungsbeispiel keine Beschränkung der Erfindung in der Art dar, dass Abwandlungen und Ergänzungen im Umfang der vorliegenden Erfindung liegen.

Dabei zeigen:
Fig. 1 eine Aufsicht auf ein Grundgehäuse eines Dreiweghahns;
Fig. 2 einen Querschnitt durch das Grundgehäuse aus Fig. 1 in Richtung der B-B-Schnittlinie;
Fig. 3 einen Querschnitt durch das Grundgehäuse aus Fig. 1 in Richtung der A-A-Schnittlinie;
Fig. 4 eine Frontalansicht des Grundgehäuses aus Fig. 1;
Fig. 5 eine Detaildarstellung der radial umlaufenden Aussparung der Stellgliedaufnahme des Grundgehäuses;
Fig. 6 ein Stellglied eines erfindungsgemäßen Mehrwegehahns;
Fig. 7 einen Teilquerschnitt durch das Grundgehäuse aus Fig. 1 in Richtung der A-A- Schnittlinie mit eingepresstem Stellglied.

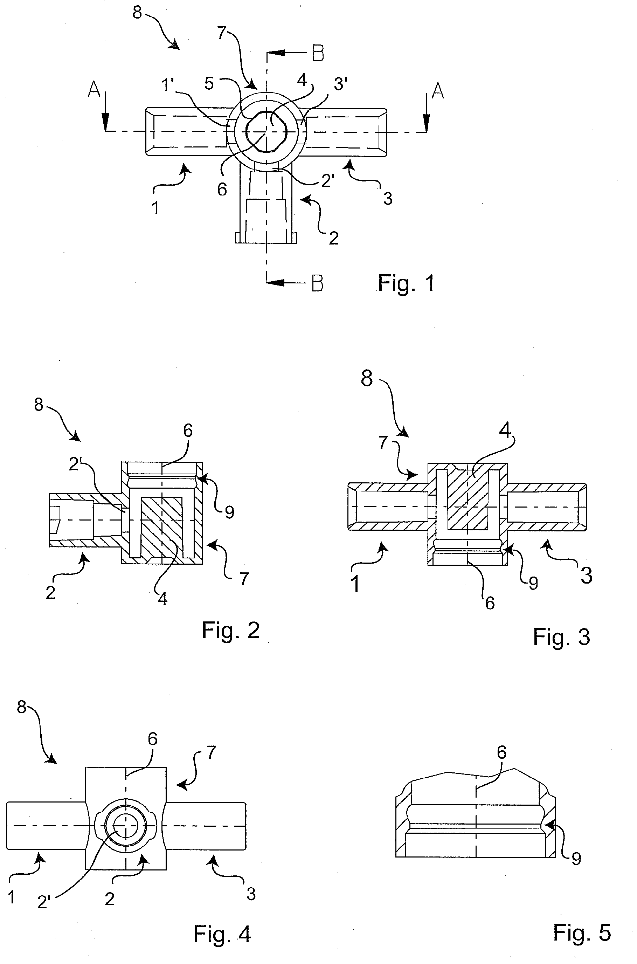

Das Ausführungsbeispiel gemäß Figur 1 zeigt das Grundgehäuse 8 mit drei Anschlussstellen 1, 2, 3 und damit korrespondierenden Durchlassöffnungen 1', 2', 3' in der Stellgliedaufnahme 7. In der Stellgliedaufnahme 7 ist um eine zentrale Achse 6 ein Zapfen 4 mit vier Abflachungen 5 angeordnet. Die unterbrochenen Linien markieren die Schnittlinien A-A und B-B für die Querschnitte in den Figuren 2 und 3.

So zeigt Figur 2 einen Querschnitt des Grundgehäuses in Richtung der B-B-Schnittlinie. In der Stellgliedaufnahme 7 ist um die zentrale Achse 6 der Zapfen 4 angeordnet. Die Anschlussstelle 2 ist mit der Stellgliedaufnahme verbunden, wobei die Durchlassöffnung 2' gebildet wird. Die Innenseite der Stellgliedaufnahme 7 weist eine radial umlaufende Aussparung 9 auf, wobei die Aussparung 9 so gestaltet ist, dass sie unterschiedliche Tiefen aufweist.

Figur 3 zeigt einen Querschnitt des Grundgehäuses gemäß Figur 2 in Richtung der in Figur 1 markierten A-A- Schnittlinie. Die beiden Anschlussstellen 1 und 3 sind mit der Stellgliedaufnahme 7 verbunden, wobei die Durchlassöffnungen 1' und 3' (Figur 1) gebildet werden. Die Innenseite der Stellgliedaufnahme 7 weist eine radial umlaufende Aussparung 9 auf, wobei die Aussparung 9 so gestaltet ist, dass sie unterschiedliche Tiefen aufweist.

Gemäß der vorliegenden Ausführungsbeispiels sind insbesondere die innenliegenden Oberflächen des Dreiwegehahns mit einem entsprechenden, im wesentlichen weichmacherfreien Material, wie beispielsweise einem Copolyester versehen. Dies kann entweder dadurch erfolgen, dass die Oberflächen entsprechend beschichtet werden oder aber auch dadurch, dass das entsprechende Bauteil aus diesem Material hergestellt wird.

Figur 4 zeigt eine Seitenansicht des Grundgehäuses von vorn entsprechend der Figur 1. Die Anschlussstellen 1, 2, 3 sind mit der Stellgliedaufnahme 7 verbunden. In der Mitte dieser Verbindungen befinden sich die entsprechenden Durchlassöffnungen, wie die Durchlassöffnung 2' in der Anschlussstelle 2.

Figur 5 zeigt einen Teil der Stellgliedaufnahme 7 mit der umlaufenden Aussparung 9 an ihrer Innenseite. Die Aussparung weist verschiedene Tiefen auf, so dass eine Abfolge von Vertiefungen und Auswölbungen gebildet wird.

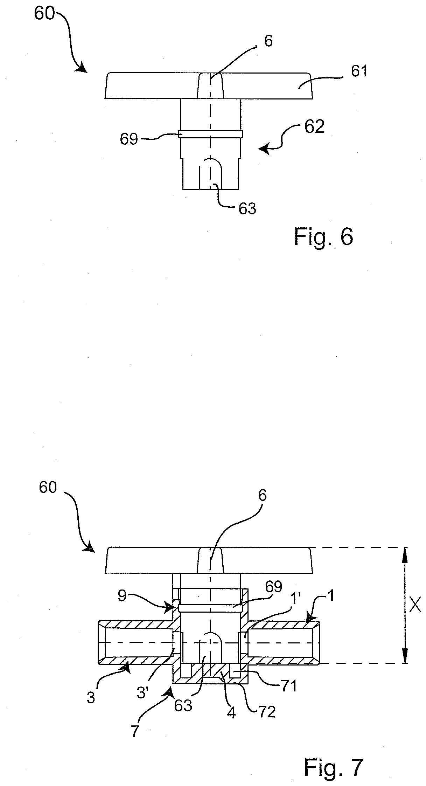

Figur 6 zeigt eine Ausführungsform eines Stellglied für die Verwendung in Verbindung mit dem Grundgehäuse gemäß Figur 1. Das Stellglied weist ein Bedienelement 61 in Form eines Hahnes mit drei Stellhebeln auf, welches fest mit einem hohlzylindrischen Abschnitt 62 verbunden ist. Der hohlzylindrische Abschnitt ist konzentrisch um eine zentrale Achse 62 angeordnet. An dem Bedienelement gegenüberliegendem Ende des hohlzylindrischen Abschnitts sind torförmige Durchlassöffnungen 63 ausgebildet. Die Zahl der Durchlassöffnungen 63 des Stellglieds entspricht der Zahl der Anschlussstellen des Grundgehäuses. Am hohlzylindrischen Abschnitt 62 des Stellglieds 60 ist eine radial umlaufende Ausformung 69 angeordnet.

Figur 7 zeigt die Verbindung des Grundgehäuses (schraffiert dargestellt) entsprechend Figur 1 mit dem Stellglied 60 entsprechend der Figur 6 als eine Ausführungsform des erfindungsgemäßen Mehrweghahns. Das Stellglied 60 ist bis zu einer vorbestimmten Einpresstiefe X in die Stellgliedaufnahme 7 des Grundgehäuses eingepresst. In dieser Position greift die Ausformung 69 des hohlzylindrischen Abschnitts des Stellglieds 60 in die Aussparung 9 an der Innenseite der Stellgliedaufnahme 7 des Grundgehäuses. Der hohlzylindrische Abschnitt des Stellglieds 60 umfasst den um eine zentrale Achse 6 angeordneten Zapfen 4 der Stellgliedaufnahme 7 und bildet so ein Gegenlager für das Stellglied 60. Der Durchfluss des Mediums im geöffneten Zustand des Mehrweghahns erfolgt von einer der Anschlussstellen 1, 3 durch die dazugehörige Durchlassöffnung des Grundgehäuses 1', 3' durch eine torförmige Durchlassöffnung des Stellglieds 63 über einen Freiraum 71 zwischen dem Stellglied 60 und dem Boden 72 der Stellgliedaufnahme 6 hin zur einer anderen Durchlassöffnung 63 des Stellglieds und durch die jeweils andere Durchlassöffnung 1', 3' des Grundgehäuses aus der jeweils anderen Anschlussstelle 1, 3 am Grundgehäuses hinaus. Die Dichtheit im oberen Bereich wird durch eine definierte Einpresstiefe X des Stellglieds 60 in den Fügerand erreicht. Im Inneren des Grundgehäuses 8 wird die Dichtheit zu den entsprechenden Durchlassöffnungen 1, 2, 3 über das konische Gegenlager, gebildet durch den Zapfen 4, sichergestellt. Die Leichtgängigkeit des Hahns, also die leichte Beweglichkeit des Stellglieds 60 in der Stellgliedaufnahme 7 wird durch vier Abflachungen 5 am Zapfen 4 sichergestellt.

## Patentansprüche

1. Verwendung eines amorphen Copolyesters zur Herstellung
eines verriegelbaren Verbindungssystems für fluiddurchströmte Bauteile für die Medizin und Medizintechnik wie insbesondere Infusions- oder Transfusionsschläuche, Mehrwegehähne, Mehrfachverteiler, Injektionsequipment wie Nadeln, Zugänge oder ähnliches und Kombinationen hiervon, mit
einem Grundgehäuse (8) mit wenigstens zwei Anschlussstellen (1, 2, 3) und einer Stellgliedaufnahme (7), welche mit den Anschlussstellen (1, 2, 3) korrespondierende Durchlassöffnungen (1', 2', 3') aufweist und in deren zentralen Achse (6) ein Zapfen (4) mit Abflachungen angeordnet ist, und,
einem Stellglied (60) mit einem Bedienelement (61), das fest mit einem hohlzylindrischen Abschnitt (62) verbunden ist, welcher konzentrisch um die zentrale Achse (6) angeordnet ist und der an dem dem Bedienelement (61) gegenüberliegenden Ende torförmige Durchlassöffnungen (63) aufweist, deren Anzahl der Zahl der Anschlussstellen (1, 2, 3) entspricht,
wobei die Anschlussstellen (1, 2, 3) eine weibliche, rohrförmige Aufnahme, welche einen inneren Aufnahmeabschnitt und einen äußeren ersten Fixierabschnitt aufweist, und/oder
ein männliches, rohrförmiges Steckelement zur Aufnahme in dem weiblichen Aufnahmeabschnitt und einem äußeren zweiten Fixierabschnitt, welcher mit dem ersten Fixierabschnitt zur Fixierung des Verbindungssystems zusammen wirkt, aufweisen,
**dadurch gekennzeichnet, dass**
der Zapfen (4) sich vom Boden (72) der Stellgliedaufnahme (7) über die Durchlassöffnungen (1', 2', 3') des Grundgehäuses (8) erstreckt und eine Gegenlager für das Stellglied (60) bildet und
der amorphe Copolyester frei von Weichmachern, insbesondere frei von Bisphenol-A ist.

2. Verriegelbares Verbindungssystem für fluiddurchströmte Bauteile für die Medizin und Medizintechnik, wie insbesondere Infusions- oder Transfusionsschläuche, Mehrwegehähne, Mehrfachverteiler, Injektionsequipment: wie Nadeln, Zugänge oder ähnliches und Kombinationen hiervon, mit
einem Grundgehäuse mit wenigstens zwei Anschlussstellen (1, 2, 3) und einer Stellgliedaufnahme (7), welche mit den Anschlussstellen (1, 2, 3) korrespondierende Durchlassöffnungen (1', 2', 3') aufweist und in deren zentralen Achse ein Zapfen (4) mit Abflachungen angeordnet ist,
einem Stellglied (60) mit einem Bedienelement (61), das fest mit einem hohlzylindrischen Abschnitt (62) verbunden ist, welcher konzentrisch um die zentrale Achse (6) angeordnet ist und der an dem dem Bedienelement (61) gegenüberliegenden Ende torförmige Durchlassöffnungen (63) aufweist, deren Anzahl der Zahl der Anschlussstellen (1, 2, 3) entspricht
wobei die Anschlussstellen (1, 2, 3) eine weibliche, rohrförmige Aufnahme, welche einen inneren Aufnahmeabschnitt und einen äußeren ersten Fixierabschnitt aufweist, und/ oder
eine männliches, rohrförmiges Steckelement zur Aufnahme in dem weiblichen Aufnahmeabschnitt und einem äußeren zweiten Fixierabschnitt, welcher mit dem ersten Fixierabschnitt zur Fixierung des Verbindungssystems zusammenwirkt, aufweisen,
**dadurch gekennzeichnet, dass**
der Zapfen (4) sich vom Boden (72) der Stellgliedaufnahme (7) über die Durchlassöffnungen (1', 2', 3') des Grundgehäuses (8) erstreckt und eine Gegenlager für das Stellglied (60) bildet und die mit dem Fluid in Kontakt kommenden Oberflächen des Verbindungssystems wenigstens abschnittsweise aus einem amorphen Copolyester hergestellt werden, wobei der amorphe Copolyesrer frei von Weichmachern, insbesondere frei von Bisphenol-A ist.

3. Verbindungssystem gemäß Anspruch 2, **dadurch gekennzeichnet, dass**
neben dem Copolyester weitere Materialien velwender werden, welche aus einer Gruppe ausgewählt werden, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, Ionomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen Kombinationen hiervon und dergleichen umfasst.

4. Verbindungssystem gemäß einem der Ansprüche 2 bis 3, **dadurch gekennzeichnet, dass**
das Fluid aus einer Gruppe ausgewählt wird, welche Injektionslösungen, Infusionslösungen, Nährlösungen, Blut, Plasma, Gase, Luft, Kombinationen hieraus und dergleichen aufweist.

5. Verwendung eines Verbindungssystems gemäß einem der Ansprüche 2 bis 4 in der Medizin und/oder Medizintechnik insbesondere für die Zu- bzw. Überleitung von verschiedenen Flüssigkeiten, insbesondere für die Schwerkraftinfusion, Pumpüberleitungssysteme, Sondennahrungssysteme, Injektionen, Kombinationen hiervon und dergleichen.

## Claims

1. Use of an amorphous copolyester to manufacture
a lockable connecting system for components for medicine and medical technology through which fluids flow, such as particularly infusion or transfusion tubes, selector valves, manifolds, injection equipment such as needles, accesses or the like and combinations thereof, comprising
a basic housing (8) having at least two connection points (1, 2, 3) and an actuator receptacle (7), having outlet openings (1', 2', 3') corresponding to the connection points (1,2,3), and in the central axis (6) of which a pin (4) with flattened surfaces is arranged, ,
an actuator (60) having an operating element (61) that is connected fixedly to a hollow-cylindrical section (62), which is arranged concentrically about the central axis (6) and which has gateway-shaped outlet openings (63) on the end thereof farthest from the operating element (61), the number of outlet openings (63) being the same as the number of connection points (1, 2, 3),
wherein the connection points (1, 2, 3,) have a female, tubular receptacle that has an inner receiving section and a first outer fixing section, and/or
a male tubular plug element for insertion in the female receiving section, and a second outer fixing section that cooperates with the first fixing section for fixing the connecting system,
**characterized in that**
the pin (4) extends from the bottom (72) of the actuator receptacle (7) to the outlet openings (1', 2', 3') of the basic housing (8) and builds a counterbearing for the actuator (60) and
the amorphous copolyester contains no plasticizers, particularly contains no bisphenol-A.

2. A lockable connecting system for components through which fluids flow and that are used in the medical and medical technology fields, particularly such as infusion or transfusion tubes, selector valves, manifolds, injection equipment such as needles, accesses or the like, and combinations thereof, with
a basic housing having at least two connection points (1, 2, 3) and an actuator receptacle (7), having outlet openings (1', 2', 3') corresponding to the connection points (1, 2, 3), and in the central axis (6) of which a pin (4) with flattened surfaces is arranged,
an actuator (60) having an operating element (61) that is connected fixedly to a hollow-cylindrical section (62), which is arranged concentrically about the central axis (6) and which has gateway-shaped outlet openings (63) on the end thereof farthest from the operating element (61), the number of outlet openings (63) being the same as the number of connection points (1, 2, 3),
wherein the connection points (1, 2, 3) have a female, tubular receptacle that has an inner receiving section and a first outer fixing section, and/or
a male tubular plug element for insertion in the female receiving section, and a second outer fixing section that cooperates with the first fixing section for fixing the connecting system,
**characterized in that**
the pin (4) extends from the bottom (72) of the actuator receptacle (7) to the outlet openings (1', 2', 3') of the basic housing (8) and builds a counterbearing for the actuator (60) and
at least sections of the surfaces of the connecting system that come into contact with the fluid are made from an amorphous copolyester, wherein the amorphous copolyester contains no plasticizers, particularly contains no bisphenol-A.

3. The connecting system according to claim 2, **characterized in that**
other materials are used besides the copolyester, and these are selected from a group including thermosetting and thermoplastic plastics, and particularly polyphenylene sulphide, polypropylene, poly-1-butene, polyvinyl chloride, polyvinylidene chloride, polymethyl metaacrylate, polyacrylic nitrile, polystyrene, polysulfone, polyacetal, polyvinyl alcohol, polyvinyl acetate, ionomer, fluoroplastic, polyethylene, polyamide, particularly a partially aromatic polyamide, polycarbonate, polyester, polyphenylene oxide, polysulfone, polyvinyl acetal, polyurethane, and chlorinated polyether, cellulose nitrate, cellulose acetate, cellulose ether, phenol resin, urea resin, thiourea resin, melamine resin, alkyl resin, allyl resin, silicone, polyimide, polybenzimidazole, epoxy resin, casein plastic, cross-linked polyurethane, unsaturated polyester resin, antimicrobial or antiseptic materials such as high-porosity silver, nonionically manufactured silver, silver compounds and particularly compounds that release metal ions, combinations thereof and the like.

4. The connecting system according to either of claims 2 to 3, **characterized in that**
the fluid is selected from a group that includes injection solutions, infusion solutions, nutrition solutions, blood, plasma, gases, air, combinations thereof and the like.

5. Use of a connecting system according to any of claims 2 to 4, in medicine and/or medical technology, particularly for infusing or transfusing fluids, particularly for gravity infusions, pump transfusion systems, tube-fed nutrition systems, injections, combinations thereof and the like.

## Revendications

1. Utilisation d'un copolyester amorphe en vue de la fabrication
d'un système de raccordement verrouillable pour des composants traversés par du fluide pour la médecine et la technique médicale, comme notamment des tuyaux flexibles de perfusion ou de transfusion, des clapets sélecteurs, des distributeurs multiplex, de l'équipement d'injection comme les aiguilles, les accès ou similaires et des combinaisons de ceux-ci, avec
une boîte de base (8) ayant au moins deux points de raccordement (1, 2, 3) et un logement de composant de réglage (7), qui présente des orifices de passage (1', 2', 3') correspondant avec les points de raccordement (1, 2, 3) et dans l'axe central (6) desquels est disposé un pivot (4) avec des aplatissements, et
un composant de réglage (60) avec un élément de commande (61) qui est relié fermement avec une section cylindrique creuse (62), qui est disposée concentriquement autour de l'axe central (6) et qui présente des orifices de passage en forme de porte (63) sur l'extrémité située face à l'élément de commande (61), dont le nombre correspond au nombre des points de raccordement (1, 2, 3),
les points de raccordement (1, 2, 3) présentant un logement tubulaire femelle, qui présente une section de logement interne et une première section externe de fixation, et/ou
un élément enfichable tubulaire mâle en vue du logement dans la section de logement femelle et une seconde section externe de fixation, qui agit conjointement avec la première section de fixation en vue de la fixation du système de raccordement,
**caractérisé en ce que**
le pivot (4) s'étend à partir du fond (72) du logement de composant de réglage (7) par les orifices de passage (1', 2', 3') de la boîte de base (8) et constitue un palier-support pour le composant de réglage (60) et le copolyester amorphe est exempt de plastifiants, notamment exempt de bisphénol-A.

2. Système de raccordement pour des composants traversés par du fluide pour la médecine et la technique médicale, comme notamment des tuyaux flexibles de perfusion ou de transfusion, des clapets sélecteurs, des distributeurs multiplex, de l'équipement d'injection : comme les aiguilles, les accès ou similaires et des combinaisons de ceux-ci, avec
une boîte de base (8) ayant au moins deux points de raccordement (1, 2, 3) et un logement de composant de réglage (7), qui présente des orifices de passage (1', 2', 3') correspondant avec les points de raccordement (1, 2, 3) et dans l'axe central desquels est disposé un pivot (4) avec des aplatissements,
un composant de réglage (60) avec un élément de commande (61) qui est relié fermement avec une section cylindrique creuse (62), qui est disposée concentriquement autour de l'axe central (6) et qui présente des orifices de passage en forme de porte (63) sur l'extrémité située face à l'élément de commande (61), dont le nombre correspond au nombre des points de raccordement (1, 2, 3),
les points de raccordement (1, 2, 3) présentant un logement tubulaire femelle, qui présente une section de logement interne et une première section externe de fixation, et/ou
un élément enfichable tubulaire mâle en vue du logement dans la section de logement femelle et une seconde section externe de fixation, qui agit conjointement avec la première section de fixation en vue de la fixation du système de raccordement,
**caractérisé en ce que**
le pivot (4) s'étend à partir du fond (72) du logement de composant de réglage (7) par les orifices de passage (1', 2', 3') de la boîte de base (8) et constitue un palier-support pour le composant de réglage (60) et Les surfaces du système de raccordement entrant en contact avec le fluide sont fabriquées au moins par sections en un copolyester amorphe, le copolyester amorphe étant exempt de plastifiants, notamment exempt de bisphénol-A.

3. Système de raccordement selon la revendication 2, **caractérisé en ce que**
Outre le copolyester, d'autres matériaux sont utilisés, qui sont sélectionnés à partir d'un groupe qui se compose du duroplastique et du thermoplastique, et notamment du polysulfure de phénylène, du polypropylène, du poly-1-butylène, du chlorure de polyvinyle, du chlorure de polyvinylidène, du méta-acrylate de polyméthyle, du polyacrylonitrile, du polystyrène, du polysulfone, du polyacétal, de l'alcool polyvinylique, de l'acétate polyvinylique, des ionomères, du plastique fluoré, du polyéthylène, du polyamide, notamment un polyamide aromatique partiel, du polycarbonate, du polyester, du polyphénylène-oxyde, du polysulfone, de l'acétal polyvinylique, du polyuréthane et du polyéther chloré, du nitrate de cellulose, de l'acétate de cellulose, de l'éther de cellulose, de la résine phénolique, de la résine d'urée, de la résine thio-urée, de la résine de mélamine, de la résine d'alcoyle, de la résine allylique, de la silicone, du polyimide, du polybenzimidazole, de la résine époxy, du plastique à la caséine, du polyuréthane réticulé, de la résine de polyester non saturée, des matériaux antimicrobiens ou antiseptiques, comme par exemple de l'argent hautement poreux, de l'argent fabriqué sans ions, des liaisons d'argent et notamment du nano-argent, des liaisons dégageant des ions métalliques, des combinaisons de ceux-ci et des produits similaires.

4. Système de raccordement selon une quelconque des revendications 2 à 3, **caractérisé en ce que**
le fluide est sélectionné à partir d'un groupe qui comprend des solutions d'injection, des solutions de perfusion, des bouillons de culture, du sang, du plasma, des gaz, de l'air, des combinaisons de ceux-ci et des produits similaires.

5. Utilisation d'un système de raccordement selon une quelconque des revendications 2 à 4 dans la médecine ou dans la technique médicale, notamment pour l'amenée et/ou le transfert de différents fluides, notamment pour la perfusion par gravité, les systèmes de transfert à pompe, les systèmes d'alimentation par sonde, les injections, des combinaisons de ceux-ci et des produits similaires.
